# EUROPEAN PATENT APPLICATION

(11) **EP 0 947 214 A2**
(43) Date of publication of application: **06.10.1999**
(21) Application number: 98309888.0
(22) Date of filing: 02.12.1998
(51) Int. Cl.: A61N 1/18

(54) **Skin-contact type medical treatment apparatus**

(30) Priority: 31.03.1998 JP 8623598; 10.06.1998 JP 16190098
(71) Applicant: Kabushiki Kaisha Bangahdo, Tokyo-to (JP)
(72) Inventor: Ogama, Kenji, Kanagawa-ken (JP)
(74) Representative: Jones, Michael Raymond

(57) **Abstract**

In a skin-contact type medical treatment apparatus comprising a first element formed of a synthetic resin pedestal plated with noble metals other and having a concave portion opening in the skin-contacting surface thereof, second elements of n-type semiconductor, and a rod-shaped protective resistance element, the second elements are integrally formed with conductive caps put on one end portion on the skin-contact side and the other end portion of the protective resistance element, and the protective resistance element is held in the concave portion in the first element in its standing posture. The n-type semiconductor second element is forcibly fitted in a support hole formed in the concave portion. The first and second elements are electrically connected to each other, so that the first element functions as a positive electrode, and the second element functions as a negative electrode when coming in contact with the skin.

## Description

This invention relates to a medical treatment apparatus to be attached to the skin for regaining health, and more particularly to a skin-contact type medical treatment apparatus effective for remedying unidentified complaint syndrome such as muscular stiffness.

As a domestic treatment apparatus for alleviating muscular stiffness or aches of the shoulders or other parts of the body, there have been so far proposed ion-permeating devices for curing muscular or nervous fatigue by imparting, to the skin, electric stimulation brought about by electromotive force generated by a biogalvanic battery formed with the application of a weak direct current to the body (cf. Japanese Patent Publication Gazettes SHO 61-55979, SHO 61-55980 and SHO 62-32944, and Japanese Utility Model Publication Gazette No. 3-50927).

The aforementioned ion-permeating device is used in such a manner that a semiconducting crystal electrode and a metal electrode higher in standard unipolar potential than the semiconducting crystal electrode are electrically connected to each other and brought into contact with the skin. The ion-permeating device enables muscular and nervous tissues to be continuously stimulated with electromotive force stably imparted to the skin through the electrodes without embrittling the electrodes during prolonged use. The ion-permeating device can achieve remarkably practical effects of remedying the stiffness and aches in the body parts.

It seems that the function of alleviating such stiffness and aches in the body is fulfilled by electric stimulation caused by the biogalvanic battery. Thus, it is obvious that the medical effect of the ion-permeating device can be heightened by enlarging the current from the biogalvanic battery.

As it is, when the muscular and nervous tissues are physiologically activated by the biogalvanic battery, impedance in the skin between the electrodes of the biogalvanic battery is remarkably decreased. As a result, the current flowing in the skin is increased in quantity to impart an excessive stimulation to the skin tissues, thus possibly suffering skin damages.

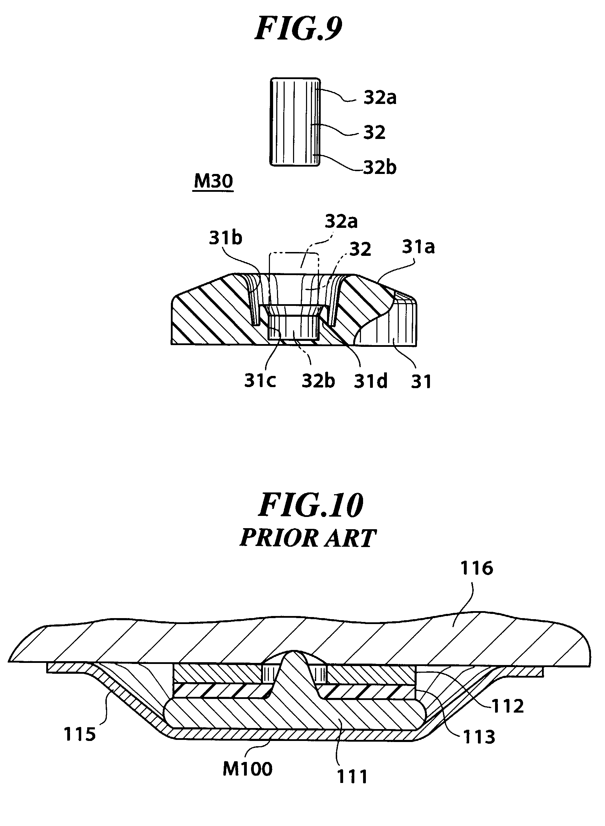

For solving such a problem, there is proposed a skin-contact type medical treatment apparatus M100 as shown in FIG. 10, in which a first element 111 serving as a metal positive electrode and a second element 112 serving as a semiconductor negative electrode are electrically connected to each other through a protective resistance element 113 (Japanese Patent Application Public Disclosure No. HEI 8-173554(A)).

Now, the prior art skin-contact type medical treatment apparatus M100 mentioned above will described in detail with reference to FIG. 10.

The first element 111 which is provided at its center with a protrusion is formed by plating a disk of copper with gold. This first element functions as a positive electrode of the biogalvanic battery when being in contact with the skin.

The second element 112 is made by oxidizing the surface of a zinc annular plate having a center hole so as to form an n-type semiconductor of zinc oxide. This second element functions as a negative electrode when being in contact with the skin.

The protective resistance element 113 is made in such a manner that epoxy synthetic resin having carbon powder dispersed therein is uniformly applied to the surface (upper side in FIG. 10) of the first element 111 and dried to be formed into a resistance layer. The protective resistance element 113 is adhered to the upper surface of the second element 112. An external circuit acting as the biogalvanic battery of the skin-contact type medical treatment apparatus is electrically connected to the protective resistance element 113.

Next, a method of producing the skin-contact type medical treatment apparatus will be explained. First, the epoxy synthetic resin to be used as the main component of the protective resistance element 113 is uniformly applied to the flat upper surface of the disk-shaped first element 111, and then, the second element 112 is placed on the flat upper surface of the first element before the epoxy synthetic resin becomes completely dry. Consequently, the first element 111 and second element 112 are superposed upon each other with the protective resistance element 113 interposed therebetween in such a manner that the center protrusion of the first element protrudes upward through the center hole in the second element. In the drawing, reference numeral 115 denotes adhesive cloth, and 116 denotes the skin.

The conventional skin-contact type medical treatment apparatus as shown in FIG. 10 brings about the intended effect of remedying unidentified complaint syndrome such as shoulder or muscular stiffness and lumbago. However, the conventional skin-contact type medical treatment apparatus is calls for the work of uniformly applying epoxy synthetic resin to form the protective resistance element in the manufacturing process. The work of producing the conventional apparatus requires not only much skills in uniformly applying the epoxy synthetic resin, but also much time and labor. Furthermore, the conventional apparatus is disadvantageous in that the producing method therefor becomes complicated because it needs the processes of putting the second element on the first element and then drying the epoxy synthetic resin applied to the first element. Thus, the conventional apparatus entails the aforenoted serious problems so that the medical treatment apparatus cannot be mass-produced uniformly in quality at a low cost with high efficiency. From the point of view of this fact, there is room for improving the conventional apparatus.

An object of the present invention is to provide a skin-contact type medical treatment apparatus having uniform quality and capable of being mass-produced at a low cost without requiring special skills in manufacture.

To attain the object described above according to the present invention, there is provided a skin-contact type medical treatment apparatus comprising a first element of metal functioning as a positive electrode, second elements of n-type semiconductor having a skin-contact side functioning as a negative electrode, and a protective resistance element having a voltage controlling function, through which the first and second elements are electrically connected to each other, wherein the first element is formed of a pedestal having a concave portion opening on its skin-contact side. The protective resistance element is formed in the shape of a rod. One of the aforementioned second elements is disposed on one end portion on the skin-contact side of the protective resistance element, and the other second element is disposed on the other end portion of the protective resistance element. The protective resistance element is seated in the aforementioned concave portion in its standing posture.

In another skin-contact type medical treatment apparatus according to this invention which comprises a first element functioning as a positive electrode, and a second element having a skin-contact side functioning as a negative electrode and electrically connected to the first element, the first element is formed of a pedestal having a concave portion opening on its skin-contact side. The concave portion is provided in its bottom with a support hole. The second element is formed of a rod of n-type semiconductor having a voltage controlling function. The second element is steadily fitted and held in position in the support hole in the concave portion.

According to the present invention, since the works of coating and drying synthetic resin for forming the protective resistance element as touched upon above are not required for manufacturing the medical treatment apparatus of the present invention, the manufacturing process for the medical treatment apparatus can be simplified and carried out easily without requiring special skills. Consequently, the apparatus of the invention can be easily and speedily assembled and produced at a low cost and uniformly in quality with high efficiency.

Furthermore, according to the present invention, since the first element is made of a metal-plated synthetic resin pedestal and the second element is formed on one end portion of the protective resistance element, the treatment apparatus can be mass-produced at a low cost.

One way of carrying out the invention is described in detail below with reference to drawings which illustrate only one specific embodiment, in which:-
FIG. 1 is a sectional view showing a skin-contact type medical treatment apparatus according to this invention in use;
FIG. 2 is a sectional view taken along line II-II in FIG. 1;
FIG. 3 is a partially cutaway front view showing the medical treatment apparatus of the invention in the exploded state;
FIG. 4 is an explanatory diagram showing the operating principle of the medical treatment apparatus of the invention;
FIG. 5 is a partially cutaway front view showing the second embodiment of the medical treatment apparatus according to the invention in the exploded state;
FIG. 6 is a sectional view taken along line VI-VI in FIG. 5;
FIG. 7 is a sectional view of the third embodiment of the treatment apparatus according to the invention in use;
FIG. 8 is a partially cutaway front view showing the medical treatment apparatus of FIG. 7 in the exploded state;
FIG. 9 is a partially cutaway front view of the fourth embodiment of the treatment apparatus of the invention in the exploded state; and
FIG. 10 is a cross sectional view showing a conventional skin-contact type medical treatment apparatus in use.

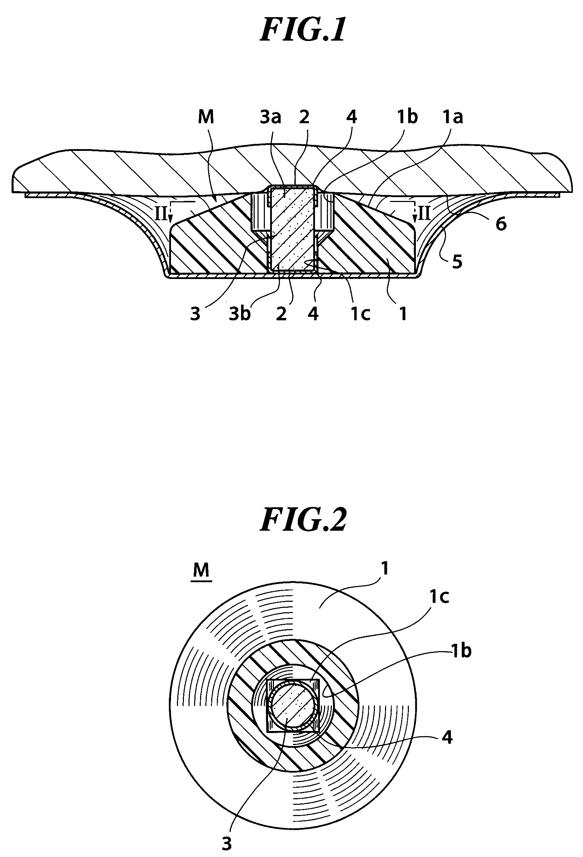

The first embodiment of the skin-contact type medical treatment apparatus according to this invention will be described hereinafter with reference to FIG. 1 through FIG. 3.

The illustrated skin-contact type medical treatment apparatus M comprises a pedestal 1 as a first element, second elements 2, and a protective resistance element 3 integrally connected to the second elements 2.

The pedestal 1 of the first element functions as a positive electrode of a biogalvanic battery when coming into contact with the skin, and has its main body molded of synthetic resin. The exterior surface of the synthetic resin pedestal 1 is entirely plated with conductive metal having higher standard unipolar potential than that of an n-type semiconductor crystal, which is typified by noble metals such as gold and platinum, or other metal alloys in order to possess electrical conductivity. The pedestal 1 has a concave portion 1b having an opening in the skin-contacting surface 1a for coming in contact with the skin. The pedestal 1 is provided in its inner bottom portion with a support hole 1c for supporting the protective resistance element 3. In the embodiment shown in FIG. 1, the support hole 1c is a through hole piercing the pedestal 1 in the thickness direction thereof.

Incidentally, the material with which the pedestal is plated is by no means limited merely to the noble metals or other metal alloys as noted above.

The second element 2 is made of material having the properties of n-type semiconductor so as to function as a negative electrode of the biogalvanic battery when being in contact with the skin.

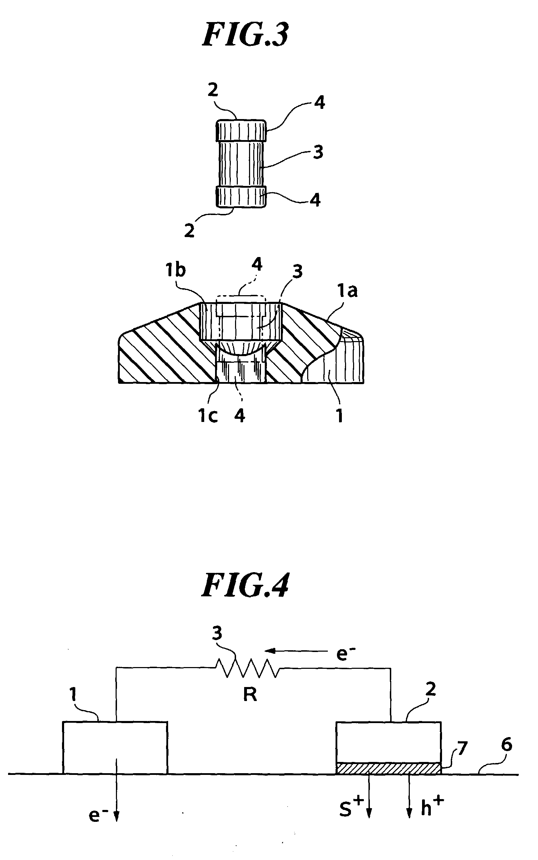

The protective resistance element 3 is shaped in a rod which protrudes upward so that one end 3a (upper extremity in FIG. 1) of the rod produces a pointillage effect when bringing the protective resistance element into press contact with the skin 6, as will be described later. In the embodiment shown in FIGS. 1 and 3, a ceramic rod constituting the protective resistance element 3 is coated with carbon and provided on its both ends 3a and 3b with conductive caps 4 (e.g. galvanized iron caps).

The second elements 2 are formed by the conductive caps 4 mounted on the both ends 3a and 3b of the protective resistance element. The method for forming the second elements 2 will be described now. The second element 2 is made by plating the surface of the conductive cap 4 with zinc and treating the plated surface of the cap with acid or heat to form oxide semiconductor layer on the cap.

The n-type semiconductor protective resistance element 3 having the ends 3a and 3b covered with the conductive caps 4 is similar in structure to, for example, a resistor usually used as an electronic part. That is, the conductive cap 4 is equivalent in structure to a cap-shaped resistance element of zinc oxide. Accordingly, the second elements 2 integrally connected to the protective resistance element 3 can easily be produced in large quantities at a low cost.

The protective resistance element 3 is seated in the concave portion 1b in the pedestal 1 in its standing posture, and securely held by tightly fitting the basal end part (lower part) 3b thereof opposite to the skin-contact end part of the protective resistance element into the support hole 1c formed in the concave portion 1b. Thus, the second element 2 formed on the basal end part 3b comes in contact with the pedestal 1. Consequently, the second element 2 on the skin-contact side which functions as the negative electrode is electrically connected to the pedestal 1 serving as the positive electrode through the protective resistance element 3 integrally connected to the second element.

The skin-contact type medical treatment apparatus M of the invention is produced by forcibly inserting the basal end part (lower end) 3b of the protective resistance element 3 into the support hole 1c formed in the concave portion of the pedestal 1 with its skin-contacting surface 1a upward.

The skin-contact type medical treatment apparatus having the aforementioned structure in use is attached to a body part suffering stiffness or aches by use of adhesive cloth 5, as shown in FIG. 1 by way of example. At the time when the apparatus is attached to the skin 6, the skin-contact end part (upper part in FIG. 1) 3a of the rod-shaped protective resistance element 3 is brought in press contact with the skin 6 by forcibly pressing the skin-contacting surface 1a of the pedestal 1 against the skin 6, thus to achieve the pointillage effect.

Next, the operating principle of the skin-contact type medical treatment apparatus M of the invention will be described with reference to FIG. 4.

Electrons e ⁻ which are emitted from the second element 2 of n-type semiconductor into the pedestal 1 through a resistance R (protective resistance element 3) are released into the skin 6, consequently bringing about a reduction effect.

On the other hand, the second element 2 of n-type semiconductor lacking the electrons e ⁻ produces holes h ⁺ which concentrate in the skin due to an internal electric field of a Schottky barrier generated in the surface layer of the second element being in contact with the skin. As a result, the n-type semiconductor 2 is ionized to allow the holes to permeate the skin along with positive ions S ⁺ due to the internal electric field. Consequently, the skin 6 undergoes an oxidizing action brought about by the holes. The oxidizing action is stably continued owing to the Schottky barrier formed in the surface layer of the second element being in contact with the skin, which functions to prevent electrons and positive ions from flowing from the skin into the second element, while generating electromotive force for a long time.

Reference numeral 7 denotes a high electrical field region.

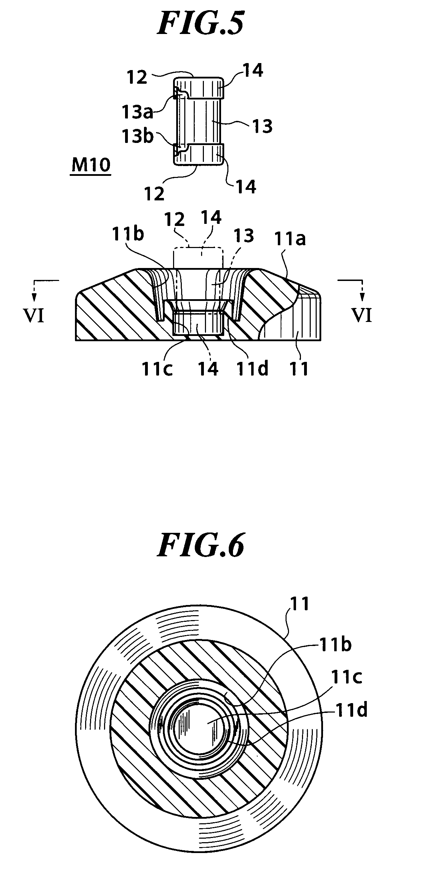

The second embodiment of this invention will be described hereinafter with reference to FIG. 5 and FIG. 6.

The illustrated skin-contact type medical treatment apparatus M10 comprises a pedestal 11 as a first element, a second element 12, and a protective resistance element 13 integrally connected to the second element. The medical treatment apparatus of this embodiment is substantially identical in structure with the foregoing embodiment.

Accordingly, the different structure between the skin-contact type medical treatment apparatus M10 and the skin-contact type medical treatment apparatus M will be touched on here, and the description of the identical or equal component parts of them will be omitted to avoid repetition.

In the skin-contact type medical treatment apparatus M10, a concave portion 11b in the pedestal 11 is formed in a substantially U shape with a bottom, which opens on the skin-contact side. In the bottom of the concave portion 11b, there is formed a support hole 11c for securing the protective resistance element 13. The support hole 11c on the bottom of the concave portion 11b is encircled by a support frame 11d formed on the concave bottom. The protective resistance element 13 is placed within the concave portion 11b in the pedestal 11 in its standing posture, and securely held by closely fitting the basal end part (lower part) 13b thereof opposite to the skin-contact end part of the protective resistance element into the support hole 1c formed in the concave portion 11b. Since the support hole 11c is formed within the support frame 11d, the support frame is elastically deformed when inserting the protective resistance element 13 thereinto, so that the protective resistance element can be forcibly inserted thereinto by small force with ease. Thus, the work of fitting the protective resistance element into the support frame becomes easy.

Reference numeral 14 denotes a conductive cap placed on each of the end portions 13a and 13b of the protective resistance element 13.

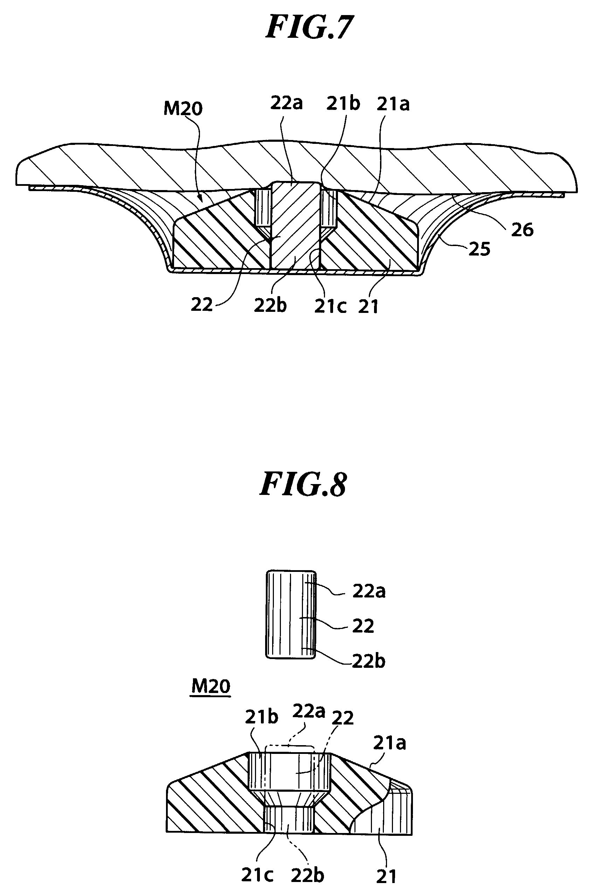

Next, the third embodiment of this invention will be described with reference to FIG. 7 and FIG. 8.

The illustrated skin-contact type medical treatment apparatus M20 comprises a pedestal 21 as a first element functioning as a positive electrode, a second element 22 of n-type semiconductor having at least a skin-contact side functioning as a negative electrode. The second element 22 may be endowed with a voltage controlling function. Both the elements are electrically connected to each other. In particular, the skin-contact type medical treatment apparatus M20 is featured by omitting the protective resistance element 3 as found in the skin-contact type medical treatment apparatus M of the foregoing embodiment. Further, the second element 22 is made of oxide semiconductor. That is, the second element 22 is made by plating the entire surface of an iron rod or the like with zinc, and then, treating the plated surface of the iron rod with acid or heat to form a layer of zinc oxide (ZnO) on the surface of the iron rod. Thus, the desired second element 22 of oxide semiconductor can be obtained. As an alternative method, the second element 22 may be formed by plating an iron rod with tin instead of zinc to form a layer of tin oxide (SnO) on the surface of the iron rod. Consequently, the desired second element 22 of oxide semiconductor can be obtained in the similar manner. In replacement of the aforementioned zinc oxide or tin oxide, there may be used indium oxide (In ₂ O ₃ ), antimony oxide (Sb ₂ O ₅), oxidation-defective aluminum oxide (Al ₂ O _{3 - x}), oxidation-defective vanadium oxide (VO _{5 - x}), and so on.

Also in the skin-contact type medical treatment apparatus M20, the same pedestal 1 in the skin-contact type medical treatment apparatus M of the foregoing embodiment is used.

The second element 22 is placed in the concave portion 21b in the pedestal 21 in its standing posture in such a manner that the basal end part (lower part) 22b thereof is fitted into the support hole 21c formed in the concave portion 21b so as to come into contact with the pedestal 21. Thus, the second element 22 having its skin-contact side (upper part) functioning as a negative electrode is electrically connected to the pedestal serving as a positive electrode. The upper end part 22a of the second element 22 protrudes to produce a pointillage effect when being pressed against the skin 26. Reference numeral 21a denotes the skin-contact surface of the pedestal 21, and 25 denotes adhesive cloth.

Next, the fourth embodiment of the invention will be described with reference to FIG. 9.

The illustrated skin-contact type medical treatment apparatus M30 comprises a pedestal 31 as a first element, and a second element 32, and is substantially identical in structure with the skin-contact type medical treatment apparatus M20 described above. Therefore, the different structure between the skin-contact type medical treatment apparatus M30 and the foregoing skin-contact type medical treatment apparatus M20 will be described here, and the description of the identical or equal component parts of them will be omitted to avoid repetition.

In the skin-contact type medical treatment apparatus M30, a concave portion 31b in the pedestal 31 is formed in a substantially U shape with a bottom, which opens on the skin-contact side. In the bottom of the concave portion 31b, there is formed a support hole 31c for retaining the second element 32.

The support hole 31c on the bottom of the concave portion 31b is encircled by a support frame 31d formed on the concave bottom. The second element 32 is placed in the concave portion 31b in the pedestal 31 in its standing posture in such a manner that the lower end part 32b is fitted into the support hole 31c formed in the concave portion 3lb. Reference numeral 32a denotes the upper end portion of the second element 32.

The main bodies of the first elements 1, 11, 21 and 31 shown in FIGS. 1, 5, 7 and 9 each are formed of a pedestal of synthetic resin, whose entire surface is plated with noble metals such as gold and platinum or alloys of such noble metals to possess electrical conductivity.

However, the first elements 1, 11, 21 and 31 per se may be entirely made of electrically conductive noble metals. That is, the first element in the medical treatment apparatus of the invention should not be understood as being limited merely to a pedestal covered with conductive materials.

## Claims

1. A skin-contact type medical treatment apparatus comprising a first element of metal functioning as a positive electrode, second elements of n-type semiconductor having a skin-contact side functioning as a negative electrode, and a protective resistance element having a voltage controlling function, said first and second elements being electrically connected to each other through said protective resistance element, said first element being formed of a pedestal having a concave portion opening on its skin-contact side, said protective resistance element being formed in a rod, one of said second elements being placed on one end portion on said skin-contact side of said protective resistance element, and the other second element being placed on the other end portion of said protective resistance element, said protective resistance element being placed within said concave portion in its standing posture.

2. A skin-contact type medical treatment apparatus according to claim 1, wherein said pedestal is plated with conductive metal having higher standard unipolar potential than that of an n-type semiconductor crystal.

3. A skin-contact type medical treatment apparatus according to claim 1, wherein said pedestal is plated with noble metals or alloys thereof.

4. A skin-contact type medical treatment apparatus according to any of claims 1 to 3, wherein said pedestal is provided in its inner bottom with a support hole into which said other end portion of said protective resistance element is forcibly fitted to secure said protective resistance element.

5. A skin-contact type medical treatment apparatus according to any of claims 1 to 4, wherein said second elements are integrally connected to said protective resistance element.

6. A skin-contact type medical treatment apparatus according to any of claims 1 to 5, wherein said protective resistance has both end portions covered with conductive caps by which said second elements are formed.

7. A skin-contact type medical treatment apparatus according to claim 1, wherein said first element is formed of conductive materials.

8. A skin-contact type medical treatment apparatus comprising a first element functioning as a positive electrode, and a second element having a skin-contact side functioning as a negative electrode and electrically connected to said first element, said first element being formed of a pedestal having a concave portion opening on its skin-contact side, said concave portion being provided in its bottom with a support hole, said second element being formed of a rod of n-type semiconductor material having a voltage controlling function.

9. A skin-contact type medical treatment apparatus according to claim 8, wherein said second element is secured in said support hole in said concave portion in its standing posture.

10. A skin-contact type medical treatment apparatus according to any of claims 1 to 9, wherein said first element is formed of a pedestal of synthetic resin, said pedestal being plated with conductive metal having higher standard unipolar potential than that of an n-type semiconductor crystal.

11. A skin-contact type medical treatment apparatus according to claim 8 or claim 9, wherein said first element is made of conductive materials.
